Europäisches Patentamt

European Patent Office

Office européen des brevets

(19)

(11) Numéro de publication : **0 011 562**
**B2**

(12) **NOUVEAU FASCICULE DE BREVET EUROPÉEN**

(45) Date de publication du nouveau fascicule du brevet :
28.03.90

(51) Int. Cl.[5] : **C 12 N 15/00// C12N9/88**

(21) Numéro de dépôt : 79400853.2

(22) Date de dépôt : 13.11.79

(54) Saccharomyces cer. transformés par un plasmide.

(30) Priorité : 14.11.78 FR 7832100

(43) Date de publication de la demande :
28.05.80 Bulletin 80/11

(45) Mention de la délivrance du brevet :
18.03.81 Bulletin 81/11

(45) Mention de la décision concernant l'opposition :
28.03.90 Bulletin 90/13

(84) Etats contractants désignés :
BE CH DE GB IT NL

(56) Documents cités :
CHEMICAL ABSTRACTS, vol. 86, no. 13, 28 mars 1977, page 242, no. 86012r, Columbus, Ohio, USA, C.P. HOLLENBERG et al.: "Synthesis of high molecular weight polypeptides in Escherichia coli minicells directed by cloned Saccharomyces cerevisiae 2-um DNA"
CHEMICAL ABSTRACTS, vol. 87, no. 11, 12 septembre 1977, page 282, no. 81110g, Columbus, Ohio, USA, M. GUERINEAU et al.: "Structure and genetics of the 2-um circular DNA in yeast"
CHEMICAL ABSTRACTS, vol. 90, no. 15, 9 avril 1979, page 319, no. 117850b, Columbus, Ohio, USA, M.L. BACH et al.: "Evidence for transcriptional regulation of orotidine-5'-phosphate decarboxylase in yeast by hybridization of mRNA to the yeast structural gene cloned in Escherichia coli"
CHEMICAL ABSTRACTS, vol. 91, no. 5, 30 juillet 1979, page 304, no. 35566v, Columbus, Ohio, USA, C. GERBAUD et al.: "High frequency of yeast transformation by plasmids carrying part or antire 2-um yeast plasmid"
Beggs, J.D., Nature, vol. 275, 14 sept. 1978, pp. 104-109

(73) Titulaire : ANVAR Agence Nationale de Valorisation de la Recherche
43, rue Caumartin
F-75436 Paris Cédex 09 (FR)

(72) Inventeur : Aigle, Michel
151, Route Burkel
67400 Illkirch-Graffemstaden (FR)
Inventeur : Blanc, Hughes
71,Av. du Général Leclerc Résidence Aquitaine II
F-92100 Boulogne Billancourt (FR)
Inventeur : Fournier, Philippe
19 bis, Bld de la République
F-78000 Versailles (FR)
Inventeur : Gerbaud, Claude
3, Allée Dunant
93220 Gagny (FR)
Inventeur : Guerineau, Michel
45, rue St-Placide
F-75006 Paris (FR)
Inventeur : Heslot, Henri, Prof.
29, rue Rousselet
Paris 7 (FR)
Inventeur : Lacroute, François, Prof.
20, rue de Copenhague
67000 Strasbourg (FR)

(74) Mandataire : Martin, Jean-Jacques et al
Cabinet REGIMBEAU 26, Avenue Kléber
F-75116 Paris (FR)

EP 0 011 562 B2

Struhl, K. et coll., 9th International Conference on Yeast Genetics and Molecular Biology, June 26th-June 30th, 1978

Bach, M.L. et coll., Ibidem, abstract 352

Carbon, J. et coll., Ibidem, abstract 23

"Proceedings of the Third International Symposium on Genetics of Industrial Microorganisms", held at the University of Wisconsin, Madison, 4-9 June 1978, ed. by Sebeck & Laskin, Am. Soc. Microbiology, 1979, pp. 36-43, "Yeast Transformation: a New Approach for the Cloning of Eucaryotic Genes" (A. Hinnen et al)

## Description

La présente invention concerne une nouvelle souche de levures comportant des plasmides hybrides.

Récemment des procédés mettant en œuvre des plasmides bactériens ont permis d'obtenir des souches de bactéries produisant des peptides et des protéines très intéressants sur le plan industriel comme la somatostatine, l'insuline et l'ovalbumine.

Toutefois, ces systèmes bactériens, compte tenu des différences qui existent dans les systèmes de transcription et de traduction eucaryotes et procaryotes, sont soumis à de sérieuses limitations. En outre, un certain nombre de gènes eucaryotes sont discontinus et présentent des insertions (jusqu'à 7 pour l'ovalbumine de poule). Cette situation explique que de tels gènes ne peuvent être correctement traduits par un procaryote, à moins évidemment de construire un gène sans insertion.

Il est donc clair que, dans ce domaine, les levures qui sont des microorganismes eucaryotes offrent la possibilité de s'affranchir de ces contraintes et présentent donc un grand intérêt potentiel pour de multiples applications pratiques.

Toutefois, jusqu'à une date récente, on ignorait comment faire rentrer un acide désoxyribonucléique (ADN) exogène dans une levure et obtenir qu'il s'y maintienne sous forme stable et s'y exprime.

Cet obstacle a récemment été levé par les travaux de A. Hinnen, J. B. Hicks et G. R. Finck en utilisant un plasmide bactérien portant le gène LEU$_2$$^+$ de la levure (vecteur). Les clones de levures obtenus par traitement à l'aide de ce plasmide se sont avérés avoir intégré tout ou partie du vecteur sans toutefois que celui-ci se maintienne à l'état libre dans le cytoplasme.

Ce procédé présente toutefois le désavantage de ne pas permettre l'amplification de l'ADN d'origine exogène. On sait, en effet, que certains plasmides bactériens existent à l'état de copie multiple dans les bactéries qui les portent et, par ailleurs, on sait qu'il existe dans certaines souches de levures, en particulier Saccharomyces cerevisiae, un plasmide désigné par le sigle 2 μ en raison de sa longueur. Ce plasmide 2 μ existe au nombre de°50 à 100 exemplaires par cellule et si on ne lui connaît pas, jusqu'à présent, de fonction génétique précise, on sait qu'il est transcrit au moins en partie et qu'il peut ainsi constituer un vecteur potentiel d'un grand intérêt.

La présente invention a pour but de fournir une souche de Saccharomyces cerevisiae transformée par des vecteurs plasmidiques permettant d'introduire à volonté un gène particulier dans ladite souche.

Un autre but de l'invention est que le gène particulier ainsi introduit soit stable, puisse s'exprimer et soit amplifié.

Grâce à cette invention, on dispose ainsi d'un moyen permettant de modifier les propriétés d'une levure de façon prédéterminée et ce dans des proportions très supérieures aux techniques de mutations connues.

La présente invention concerne une souche de Saccharomyces cerevisiae transformée par un plasmide hybride comportant l'ADN d'un plasmide bactérien, tout ou partie de l'ADN du plasmide 2 μ de levure et un segment d'ADN d'environ 1,1 kb incorporant le gène URA$_3$$^+$ de levure qui est limité par 2 sites de restriction Hind III.

Il peut être utile de rappeler que le plasmide 2 μ de levure est connu et une étude très complète peut en être trouvée dans « Viruses and Plasmid in Fungi » éditeur Paul A. Lemke, cet ouvrage pourra également être utilisé comme référence pour la définition de certains termes dont la définition complète ne serait pas donnée dans la présente description.

Le gène URA$_3$$^+$ est le gène codant pour l'orotidine-5'-phosphate-décarboxylase, en son absence la levure ne peut se développer que sur un milieu contenant de l'uracil. La présence ou l'absence de ce gène permet de « cribler » les levures en utilisant un milieu avec et sans uracil.

Dans un mode de réalisation préféré du plasmide selon la présente invention, le segment d'ADN renfermant le gène URA$_3$$^+$ de levure est inséré dans l'ADN du plasmide 2 μ de levure, en particulier le segment d'ADN renfermant le gène URA$_3$$^+$ de levure est inséré dans l'ADN du plasmide 2 μ de levure entre les sites de restriction Hind III (2) et Hind III (3) avec perte du segment correspondant de l'ADN du plasmide 2 μ de levure.

Les sites de restriction Hind III correspondent aux endroits de la molécule ADN qui sont coupés par une enzyme particulière, l'endonucléase Hind III (qui sera appelée ci-après par abréviation Hind III). Ces sites de restriction Hind III sont au nombre de 3 sur le plasmide 2 μ et sont appelés Hind III (1), Hind III (2) et Hind III (3) (qui seront appelés ci-après par abréviation H1, H2 et H3).

Dans un autre mode de réalisation des plasmides selon la présente invention, le segment d'ADN renfermant le gène URA$_3$$^+$ de levure est inséré dans l'ADN du plasmide bactérien.

Parmi les ADN de plasmide bactérien utilisables il faut citer plus particulièrement l'ADN du plasmide pCR1 et du plasmide pBR322 (Bethesda Research Laboratory Inc., in Rockville, Maryland).

Dans un mode de réalisation particulièrement intéressant de la présente invention, en particulier lorsque le segment d'ADN renfermant le gène URA$_3$$^+$ est inséré dans l'ADN du plasmide 2 μ, l'ADN du plasmide bactérien comporte l'insertion d'un ADN exogène provenant d'un organisme procaryote ou surtout d'un organisme eucaryote tel qu'une levure.

On peut également prévoir dans le cadre de la présente invention des plasmides hybrides dans lesquels le plasmide 2 μ comporte une insertion d'un ADN exogène provenant d'un organisme procaryote ou surtout eucaryote.

Les plasmides vecteurs selon la présente invention peuvent être préparés par des techniques connues.

On donnera, ci-après, une technique générale permettant de préparer deux plasmides selon la présente invention particulièrement intéressants.

Afin de faciliter la compréhension du procédé, certains des plasmides mis en œuvre dans cette technique sont représentés sur les figures annexées où :

la figure 1 représente le schéma de l'ADN du plasmide PTY39,

la figure 2 représente le schéma de l'ADN du plasmide G9,

la figure 3 représente le schéma de l'ADN du plasmide G18.

la figure 4 représente le schéma de l'ADN du plasmide pFL1,

la figure 5 représente le schéma de l'ADN du plasmide pMA1,

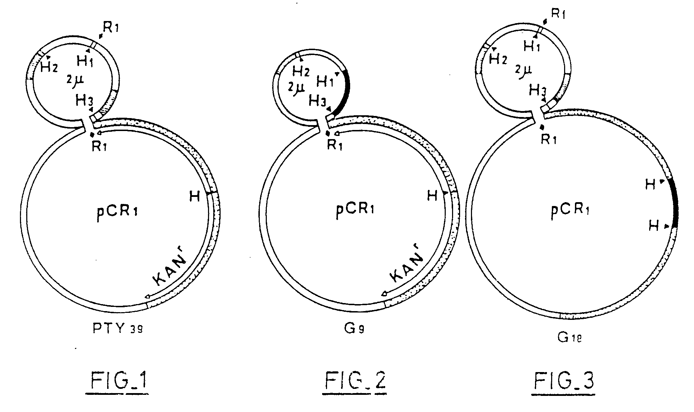

On part d'un plasmide hybride PTY39 décrit dans l'article de Hollenberg et al., Proc. Nat. Acad. Sci. USA, 73, 2072-2076, (1976). Ce plasmide est représenté à la figure 1.

Le plasmide PTY39 est constitué de deux séquences :

l'ADN du plasmide 2 μ de levure et

l'ADN bactérien de pCR1 sur lequel on a indiqué en pointillé et par les lettres KAN$^r$ le gène du plasmide qui lui permet de conférer le caractère de résistance à la kanamycine.

Le fragment d'ADN correspondant au plasmide pCR1 ne possède qu'un site de restriction Hind III désigné par H et situé dans le gène KAN$^r$.

L'ADN du plasmide 2 μ de PTY39 comporte pour sa part trois sites de restriction Hind III qui sont numérotés H1, H2 et H3 et un site de restriction Eco R1, noté R1, entre H1 et H3 en plus des sites de restriction Eco R1 de jonction, comme précédemment, la présence de ces sites de restriction Eco R1 signifie que l'ADN est coupé à ce niveau par l'endonucléase Eco R1.

Il est important de noter que le site de restriction Eco R1 est situé entre les sites de restriction Hind III (1) et Hind III (3).

Les éléments hachurés de l'ADN du plasmide 2 μ situent les séquences répétées inverses.

Ce plasmide hybride PTY39 est accumulé en un très grand nombre de copies en traitant la bactérie qui en est porteuse par le chloramphénicol.

D'un autre côté, on utilise un plasmide hybride pMB9-URA$_3$$^+$ constitué d'un plasmide bactérien pMB9 dans lequel on a inséré un fragment d'ADN de levure portant le gène URA$_3$$^+$ encadré par deux sites de restriction Hind III.

On effectue tout d'abord une digestion modérée du plasmide PTY39 par l'endonucléase Hind III.

Puis une digestion complète du plasmide pMB9-URA$_3$$^+$ par la même enzyme suivie d'une séparation des fragments de restriction par électrophorèse et on isole un fragment de 1,1 kilobase portant le gène URA$_3$$^+$.

On mélange alors le plasmide PTY39 partiellement digéré avec le fragment portant le gène URA$_3$$^+$ isolé précédemment en présence de ligase.

Puis on transforme des bactéries pyrF (c'est-à-dire affectées dans le gène de l'orotidine-5'-phosphate-décarboxylase) par l'ADN obtenu après ligation à l'étape précédente.

On sélectionne ensuite les clones se développant sur milieu minimum qui sont les Escherichia coli pyr$^+$ qui ont donc intégré les plasmides portant le gène URA$_3$$^+$.

Parmi ces Escherichia coli pyr$^+$ on sépare les clones sensibles à la kanamycine, on obtient ainsi deux types de souches :

Escherichia coli pyr$^+$ Kan$^r$ et
Escherichia coli pyr$^+$ Kan$^s$.

On extrait alors de ces deux types de souches deux types de plasmides selon l'invention :

le plasmide G9 qui confère le phénotype pyr$^+$ Kan$^r$ et
le plasmide G18 qui confère le phénotype pyr$^+$ Kan$^s$.

Les plasmides G9 et G18 sont représentés respectivement sur les figures 2 et 3.

Le plasmide G9 comporte une partie d'ADN bactérien provenant de pCR1 identique à celle que l'on trouvait dans PTY39. Quant à la partie ADN de levure, on a représenté en trait noir épais le segment d'ADN porteur de gène URA$_3$$^+$ et qui s'est fixé entre les sites de restriction H1 et H3 avec une perte du segment de plasmide 2 μ correspondant.

Il est important de noter que le remplacement du segment d'ADN du plasmide 2 μ situé entre les sites de restriction Hind III (1) et Hind III (3) par l'ADN de levure portant le gène URA$_3$$^+$ a pour effet de faire disparaître le site de restriction Eco R1 se trouvant à l'origine dans l'ADN du plasmide 2 μ entre les sites H1 et H3.

4

G18 comporte intégralement l'ADN du plasmide 2 µ comme pour PTY39, par contre le fragment d'ADN bactérien provenant de pCR1 comporte l'insertion, au niveau du site de restriction Hind III, du fragment d'ADN portant le gène $URA_3^+$ représenté en trait noir.

Les plasmides hybrides G9 et G18 ainsi obtenus peuvent être conservés tel quel ou dans une levure ou une bactérie où ils se multiplieront et pourront en être extraits à la demande.

Ces plamides peuvent être intégrés dans une levure par le procédé suivant.

On utilise une souche de levure de Saccharomyces cerevisiae $URA_3^-$, dans le cas présent et afin de mieux étudier le phénomène on utilise une levure à faible taux de reversion.

Cette souche cultivée sur milieu complet est récoltée en phase exponentielle.

Les cellules sont alors transformées en protoplastes par digestion des parois à l'aide d'hélicase en présence d'un stabilisateur osmotique.

Les protoplastes sont mis en présence de l'ADN du plasmide hybride G9 ou G18 pendant environ 10 minutes puis on mélange avec du polyéthylène glycol à 30 % et on laisse agir pendant environ 15 minutes. On centrifuge et le culot est remis en suspension dans un milieu complet contenant un stabilisant osmotique. On incube pendant 1 heure à 30 °C puis on procède à une deuxième centrifugation. Le culot est alors resuspendu dans un milieu hypertonique contenant de l'agar (3 %) maintenu en surfusion à 44 °C et 0,03 % d'extrait de levure.

L'ensemble est coulé dans des boîtes de Pétri.

Aux fins de comparaison, on traite de la même façon des levures témoins mais sans ajouter d'ADN de plasmide hybride.

Au bout de 3 à 5 jours, il apparaît de nombreuses colonies sur les boîtes correspondant aux cellules traitées par l'ADN alors qu'il n'y en a pas sur les boîtes témoins.

Les colonies ainsi obtenues sont appelées « transformants » et constituent des microorganismes entrant dans le cadre de l'invention à la fois comme source de plasmide hybride G9 ou G18 et comme moyen notamment pour la préparation de l'orotidine-5′-phosphate-décarboxylase.

En effet, on a mesuré l'activité spécifique de l'orotidine-5′-phosphate-décarboxylase dans la souche sauvage $URA_3^+$ et dans un certain nombre de transformants, c'est-à-dire de souches selon la présente invention et on a constaté que si dans la souche sauvage l'activité de l'enzyme est de deux unités, cette activité varie entre 10 et 35 chez les transformants, ce qui représente un facteur multiplicatif de 5 à 18.

Cette description permet de comprendre les fonctions des différentes parties des plasmides selon la présente invention :

le fragment $URA_3^+$ permet de « cribler » les microorganismes traités par les plasmides de l'invention

le fragment 2 µ permet d'amplifier les propriétés transférées par les plasmides de l'invention.

Les souches selon la présente invention peuvent donc être envisagées comme moyen de production de l'orotidine-5′-phosphate-décarboxylase qui est un produit utilisé dans les industries enzymatiques.

Un autre intérêt des plasmides hybrides selon la présente invention, notamment des plasmides hybrides G9 et G18, est qu'ils peuvent, dans le cadre de l'invention, être modifiés afin de servir de vecteurs pour l'introduction d'ADN exogène dans la levure qui, après transformation, font également partie de la présente invention.

Il faut remarquer, à ce propos, que dans le cas du plasmide hybride G9 ce dernier ne présente de sites de restriction Eco R1 qu'à la jonction entre l'ADN de levure et l'ADN bactérien et qu'en conséquence l'action de l'endonucléase Eco R1 conduit à la séparation de l'ADN levure et de l'ADN bactérien et qu'ainsi on peut aisément fixer sur le site Eco R1 de l'ADN levure un nouvel ADN bactérien portant par exemple un ADN exogène.

Pour ce qui concerne le plasmide hybride G18. Celui-ci présente l'avantage de posséder un ADN de plasmide 2 µ complet ce qui permet d'envisager sa réplication et son maintien dans une souche de levure par exemple de façon beaucoup plus sure que dans le cas du plasmide G9 dont l'ADN du plasmide 2 µ a été amputé d'un segment entre les sites de restriction Hind III (1) et Hind III (3). Toutefois, cet avantage doit être pondéré compte tenu du fait que G18 possède deux sites de restrictions Eco R1 qui lors de l'action de l'enzyme correspondante peuvent conduire à un fractionnement plus complexe de l'ADN que dans le cas de G9 et rendre ainsi plus difficile l'introduction d'ADN bactérien portant un ADN exogène.

Les exemples suivants sont destinés à illustrer un procédé de préparation de plasmides hybrides et de microorganismes selon la présente invention sans pour autant qu'ils puissent être considérés comme limitant la portée de ladite invention.

Exemple 1

Préparation des plasmides G9 et G18

On prépare un plasmide pMB9-$URA_3^+$ par les procédés connus (T.D. Petes et al., Gene, vol. IV, 1978, p. 37-49) par exemple en faisant digérer l'ADN d'une levure sauvage de Saccharomyces cerevisiae par l'endonucléase Hind III, et en faisant de même digérer le plasmide pMB9 (Bolivar et al. Gene 2, 75-93 (1977) par la même enzyme. Les deux produits de digestion sont ligaturés par la ligase ADN T4. On effectue ensuite le tri des plasmides obtenus en utilisant une souche de Escherichia coli URA$_3^-$ que l'on transforme par lesdits plasmides qui sont les seuls à croître sur un milieu minimum (sans uracil). On extrait alors les plasmides pMB9-URA$_3^+$ des souches Escherichia coli URA$_3^+$ sélectionnées.

20 μg du produit de digestion du plasmide pMB9-URA$_3^+$ par l'endonucléase Hind III (fourni par la firme Boehringer) sont soumis à une électrophorèse sur un gel d'agarose à 1 %.

Le fragment de 1,1 kilobase portant le gène URA$_3^+$ de la levure est récupéré à partir du gel par la méthode dite « Freeze et Squeeze » (Thuring, 1975) et par précipitation à l'éthanol.

L'ADN circulaire du plasmide PTY39 est partiellement digéré par l'endonucléase Hind III pour obtenir un maximum de molécule ne comportant qu'une seule coupure. Après chauffage à 60 °C pendant 10 minutes pour inactiver l'enzyme et après dialyse, 1 μg de l'ADN digéré de PTY39 est mélangé avec environ 0,05 μg du fragment de 1,1 kilobase portant le gène URA$_3^+$ de la levure. Les deux morceaux sont liés par de la ligase ADN T4 dans un volume de 50 μl pendant 3 minutes à 37 °C puis pendant 5 heures à 10 °C.

Le mélange de ligation (50 μl) est dilué par 900 μl d'un tampon contenant 10 mM de Tris pH 7, 10 mM de CaCl$_2$, 10 mM de MgSO$_4$. 100 μl de ce mélange dilué sont ajoutés à 200 μl de cellules d'Escherichia coli URA$_3^-$ préparé pour transformation par le procédé de Cohen et al. (1975). Le mélange de transformation est abandonné sur la glace pendant 25 minutes puis soumis pendant 3 minutes à un chauffage pulsé à 37 °C puis abandonné à la température ambiante pendant 10 minutes. 1 ml de milieu complet est alors ajouté et le mélange est agité à 37 °C pendant 1 heure. Les cellules sont alors rassemblées par centrifugation puis étalées sur un milieu minimum, c'est-à-dire un milieu M63, contenant du tryptophane. Les clones URA$_3^+$ obtenus sont alors sélectionnés en tenant compte de leur résistance à la kanamycine.

On sélectionne ainsi les clones d'Escherichia coli porteurs de G9 qui résistent à la kanamycine et les clones d'Escherichia coli porteurs de G18 qui ne résistent pas à la kanamycine.

Ces plasmides sont alors extraits.

## Exemple 2

### Préparation de levures

On prépare les souches de levures réceptrices URA$_3^-$ en les cultivant sur 500 ml de milieu complet jusqu'à une densité cellulaire d'environ $2.10^7$ cellules/ml. Les cellules sont alors lavées dans 300 ml d'eau distillée et dans le même volume de sorbitol 1,2 M. Elles sont remises en suspension dans 50 ml d'un mélange de sorbitol 1,2 M, 0,05 M phosphate-citrate pH 5,8 et d'hélicase (fourni par l'Industrie Biologique Française) qui est ajouté jusqu'à une concentration finale de 6 500 unités par ml. Les cellules sont incubées à 28 °C pendant 1 heure à 1 heure 30 sous légère agitation. Durant l'incubation la formation des sphéroplastes est suivie par une méthode optique.

Les sphéroplastes sont lavés par centrifugation à température ambiante et remis en suspension trois fois dans 150 ml de sorbitol 1,2 M puis une fois dans une solution de sorbitol 1,2 M, 10 mM de Tris pH 7,6 et 10 mM de CaCl$_2$. Les sphéroplastes sont concentrés dans le même tampon jusqu'à une densité cellulaire d'environ $10^9$ cellules/ml. L'ADN de plasmide obtenu précédemment dans CaCl$_2$ 10 mM et Tris pH 6 10 mM (environ 10 μl) est mélangé avec 0,2 ml de sphéroplastes jusqu'à une concentration de 5 à 15 μg par ml. Le mélange est abandonné à la température ambiante pendant 10 minutes puis on ajoute 2 ml de Tris 10 mM, CaCl$_2$ 10 mM dans du polyéthylène 4000 à 30 %. Après mélange, l'ensemble est abandonné 15 minutes à la température ambiante. Les sphéroplastes sont récupérés par centrifugation à 2 500 g pendant 10 minutes puis remis en suspension dans un milieu contenant 1,2 M sorbitol, 4 g/l d'extrait de levure, 6 g/l de glucose, 6 g/l de bactopeptone Difco, 10 mM de CaCl$_2$ et 10 mM de Tris pH 6 puis agité légèrement pendant 1 heure à 28 °C.

L'ensemble est centrifugé puis remis en suspension dans 0,2 ml du tampon précédent. Les échantillons sont mélangés avec 8 ml de gélose (1,2 M sorbitol, 20 g/l de glucose, 0,8 g/l de bactotryptone Difco, 0,3 g/l d'extrait de levure, 30 g/l de gélose purifiée Difco) à 44 °C et versé dans le même milieu à l'exception de la concentration en gélose qui est de 20 g/l.

Les dilutions sont étalées par le même procédé dans les mêmes milieux supplémentés avec 50 μg/l d'uracil de façon à mesurer l'efficacité de la régénération des sphéroplastes.

Les résultats obtenus sont rassemblés dans le tableau I.

## Exemple 3

On opère comme dans l'exemple 2 et on obtient les résultats rassemblés dans le tableau I.

(Voir Tableau I page 7).

Tableau I

| ADN | Ex | NOMBRE DE CELLULES PAR BOITE | NOMBRE DE CELLULES REGENEREES | POUR-CENTAGE DE REGENE-RATION | NOMBRE DE CLONES URA+ | FREQUENCE DE TRANSFOR-MATION PAR CELLULE VIABLE |
|---|---|---|---|---|---|---|
| G 18 | 2 | $2,4 \times 10^8$ | $6,4 \times 10^7$ | 26,7 | 1000 | $1,5 \times 10^{-5}$ |
| | 3 | $5,3 \times 10^6$ | $1,7 \times 10^5$ | 3,2 | 18 | $1,0 \times 10^{-4}$ |
| G 9 | 2 | $2,4 \times 10^8$ | $6,4 \times 10^7$ | 26,7 | 1500 | $2,3 \times 10^{-5}$ |
| | 3 | $5,3 \times 10^6$ | $1,7 \times 10^5$ | 3,2 | 30 | $1,8 \times 10^{-4}$ |
| Aucun | 2 | $2,4 \times 10^8$ | $6,4 \times 10^7$ | 26,7 | 0 | |
| | 3 | $5,3 \times 10^6$ | $1,7 \times 10^5$ | 3,2 | 0 | |

Exemple 4

Etude de l'activité des levures obtenues

On dose l'activité d'orotidine-5'-phosphate-décarboxylase par prélèvement des cellules en phase de croissance logarithmique sur milieu minimum et un extrait brut est préparé par le procédé de Lacroute (1962).

L'essai enzymatique est conduit selon Beckwith et al. (1962) excepté le fait que $MgCl_2$ est mis dans le mélange réactionnel. Les protéines sont dosées selon la méthode de Lowry et al. (1951) la lysozyme étant utilisée comme étalon.

Les résultats obtenus sont rassemblés dans le tableau II.

Les souches TRA (transformants) sont des souches obtenues dans les exemples 2 et 3.

L'activité spécifique pour l'orotidine-5'-phosphate-décarboxylase est exprimée en n.moles de substrat décarboxylé par minute et par milligramme de protéine.

L'augmentation de l'activité spécifique indique le rapport entre l'activité spécifique des TRA après correction à l'activité spécifique de la souche sauvage.

Tableau II

| PLAS-MIDES | SOUCHES | ACTIVITE SPECI-FIQUE | POURCENTAGE DE PROTO-TROPHES DANS LA CULTURE | ACTIVITE SPECIFIQUE CORRIGEE | AUGMENTATION D'ACTIVITE SPECIFIQUE |
|---|---|---|---|---|---|
| Aucun | Souche sauvage | 2 | 100 | 2 | 1 |
| G 9 | TRA1 | 35 | 46 | 76 | 38 |
| | TRA2 | 21 | 52 | 40 | 20 |
| | TRA3 | 33 | 44 | 75 | 38 |
| G 18 | TRA 363 | 15 | 67 | 22 | 11 |
| | TRA 366 | 10 | 43 | 23 | 12 |
| | TRA 367 | 22 | 81 | 27 | 14 |

7

## Exemple 5

De façon analogue aux exemples 1 et 2,

on prépare un plasmide pBR322 avec le fragment portant le gène URA$_3^+$ inséré au niveau du site Hind III ;

on effectue la digestion partielle du plasmide obtenu par l'enzyme Eco R1 ;

on effectue la digestion partielle du plasmide 2 μ de la souche de S. cerevisiae FI 100 ATCC 28 383 par l'enzyme Eco R1 ;

on mélange les deux produits de digestion après inhibition de l'enzyme Eco R1 et on ligature les fragments du mélange avec la ligase une nuit à 10 °C ;

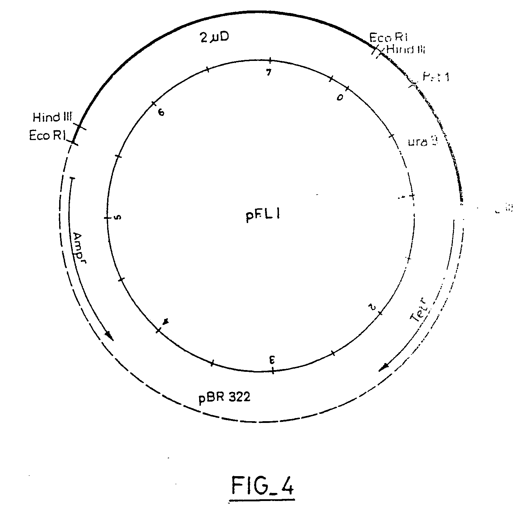

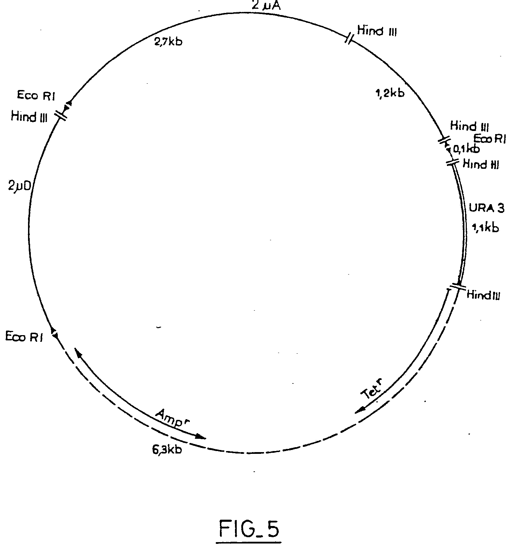

on extrait les plasmides obtenus et on transforme une souche de levure S. cerevisiae réceptrice ura$_3$. C'est-à-dire ne pouvant croître sans uracile, et on sélectionne les transformats capables de croître sur milieu sans uracile. De ces transformats URA$_3^+$ on extrait les plasmides qui peuvent, comme cela a été décrit, être utilisés pour transformer une souche E. coli URA$_3^-$, laquelle par sélection des souches URA$_3^+$ obtenues constitue un réservoir de plasmide dont la structure est donnée dans les figures 4 et 5 ci-annexées.

La figure 4 représente le plasmide pFL 1 (représenté en traits pointillés) qui comporte l'ADN du plasmide pBR 322 avec insertion de l'ADN du gène URA$_3^+$ (représenté en double trait) au niveau du site Hind III de l'ADN de pBR 322 et insertion au niveau du site Eco R1 de pBR 322 d'un fragment de l'ADN du plasmide 2 μ, le fragment 2 μD (fragment défini par rapport aux sites Eco R1).

Il a été isolé 3 autres plasmides, pFL 2, pFL 3 et pFL 4. Pour pFL 2 l'ADN du gène URA$_3^+$ a l'orientation inverse, c'est-à-dire que le site Pst 1 se trouve à 0,8 kb du site Eco RI pris comme origine. Les plasmides pFL 3 et pFL 4 ont le fragment 2 μD dans l'orientation inverse de celle observée respectivement pour pFL 1 et pFL 2. La longeur totale de ce plasmide est de 7,652 kB ;

Le plasmide pBR 322 porte un gène de résistance à l'ampicilline (Amp$^r$), gène qui code pour une pénicillinase. Dans une levure transformée par un plasmide de ce type (comme pFL 1 ou pFL 2) ce gène bactérien est exprimé et on identifie une excrétion de pénicillinase par les levures transformées, ce qui montre l'intérêt des plasmides décrits dans la présente invention pour l'excrétion de protéines (enzymes en particulier).

Un autre avantage de ces plasmides est qu'ils démontrent que l'intégralité du plasmide 2 μ n'est pas indispensable à sa réplication et à son maintien dans la levure. L'avantage de cette construction est qu'elle permet d'envisager de cloner des fragments d'ADN étrangers de grande taille puisque le plasmide récepteur est plus petit. En effet, des plasmides de trop grand taille sont plus fragiles, plus difficiles à manipuler et à extraire des bactéries ou des levures.

De façon identique, on obtient le plasmide pMA 1 représenté à la figure 5.

Ce plasmide a la même structure que les précédents mais présente l'intégralité de l'ADN du plasmide 2 μ(2 μA + 2 μD) au lieu du fragment 2 μD.

Un plasmide analogue à ceux décrits ci-dessus a été utilisé, voir Panthier J. J. et col., Comptes Rendus Acad. Sciences, Série D, 1979, Séance du 29 octobre, en y insérant l'ADN du gène lacZ d'E. coli pour transformer une souche de S. cerevisiae. On observe dans la levure transformée la présence d'une galactosidase qui est absente de la souche de départ, ceci permet de conclure que le gène bactérien lacZ est exprimé chez la levure.

## Revendications

1. Souche de Saccharomyces cerevisiae transformée par un plasmide hybride comportant l'ADN d'un plasmide bactérien, tout ou partie de l'ADN du plasmide 2 μ de levure et un segment d'ADN d'environ 1,1 kb incorporant le gène URA$_3^+$ de levure qui est limité par 2 sites de restriction Hind III.

2. Une souche selon la revendication 1, caractérisée en ce que le segment d'ADN renfermant le gène URA$_3^+$ de levure est inséré dans l'ADN du plasmide 2 μ de levure.

3. Une souche selon la revendication 2, caractérisée en ce que le segment d'ADN renfermant le gène URA$_3^+$ de levure est inséré dans l'ADN du plasmide 2 μ de levure entre les sites de restriction Hind III (2) et Hind III (3) avec perte du segment correspondant de l'ADN du plasmide 2 μ de levure.

4. Une souche selon la revendication 1, caractérisée en ce que le segment d'ADN renfermant le gène URA$_3^+$ de levure est inséré dans l'ADN du plasmide bactérien.

5. Une souche selon l'une des revendications 1 à 4, caractérisée en ce que l'ADN de plasmide bactérien est l'ADN du plasmide pCRI ou pBR322.

6. Une souche selon l'une des revendications 1 à 5, caractérisée en ce que l'ADN du plasmide 2 μ de levure est inséré dans l'ADN du plasmide bactérien entre des sites de restriction EcoRi.

## Claims

1. A strain of Saccharomyces cerevisiae transformed by a hybrid plasmid comprising the DNA of a bacterial plasmid, all or part of the DNA of the plasmid 2 μ of yeast and a DNA segment of about 1.1 kb incorporating the gene URA$_3^+$ of yeast which is limited by two Hind III restriction sites.

2. A strain according to claim 1, characterised in that the DNA segment containing the gene URA$_3^+$ of yeast is inserted in the DNA of the plasmid 2 μ of yeast.

3. A strain according to claim 2, characterised in that the DNA segment containing the gene URA$_3^+$ of yeast is inserted in the DNA of the plasmid 2 μ of yeast between the restriction sites Hind III (2) and Hind III (3) with loss of the corresponding segment of the DNA of the plasmid 2 μ of yeast.

4. A strain according to claim 1, characterised in that the DNA segment containing the gene URA$_3^+$ of yeast is inserted in the DNA of the bacterial plasmid.

5. A strain according to one of claims 1 to 4, characterised in that the DNA of the bacterial plasmid is the DNA of the plasmid pCRI or pBR322.

6. A strain according to one of claims 1 to 5, characterised in that the DNA of the plasmid 2 μ of yeast is inserted in the DNA of the bacterial plasmid between EcoRI restriction sites.


**Patentansprüche**

1. Saccharomyces cerevisiae Stamm, der durch ein hybrides Plasmid transformiert ist, das die DNA eines bakteriellen Plasmids, die ganze oder einen Teil der DNA des 2 μ-Plasmids der Hefe und ein DNA-Segment von etwa 1,1 kb, welches das URA$_3^+$-Gen der Hefe umfaßt, das durch 2 Hind III Restriktionsschnittstellen begrenzt ist, enthält.

2. Ein Stamm nach Anspruch 1, dadurch gekennzeichnet, daß das DNA-Segment, welches das URA$_3^+$-Gen der Hefe umfaßt, in die DNA des 2 μ-Plasmids der Hefe insertiert ist.

3. Ein Stamm nach Anspruch 2, dadurch gekennzeichnet, daß das DNA-Segment, welches das URA$_3^+$-Gen der Hefe umfaßt, in die DNA des 2 μ-Plasmids der Hefe zwischen die Restriktionsschnittstellen Hind III (2) und Hind III (3), unter Verlust des korrespondierenden DNA-Segments des 2 μ-Plasmids der Hefe insertiert ist.

4. Ein Stamm nach Anspruch 1, dadurch gekennzeichnet, daß das DNA-Segments, welches das URA$_3^+$-Gen der Hefe umfaßt, in die DNA des bakteriellen Plasmids insertiert ist.

5. Ein Stamm nach einem der Ansprüche 1-4, dadurch gekennzeichnet, daß die DNA des bakteriellen Plasmids die DNA des Plasmids pCRI oder pBR322 ist.

6. Ein Stamm nach einem der Ansprüche 1-5, dadurch gekennzeichnet, daß die DNA des 2 μ-Plasmids der Hefe zwischen die EcoR1-Restriktionsschnittstellen der DNA des bakteriellen Plasmids insertiert ist.

FIG_1   FIG_2   FIG_3

FIG_4

FIG_5